# EUROPÄISCHE PATENTANMELDUNG

(11) **EP 4 008 380 A1**
(43) Veröffentlichungstag der Anmeldung: **08.06.2022**
(21) Anmeldenummer: 20212250.3
(22) Anmeldetag: 07.12.2020
(51) Int. Cl.: A61M 5/32, A61M 5/31, A61M 5/50, A61M 5/28

(54) **MEDIZINISCHE SPRITZE**

(71) Anmelder: Trenta2 S.r.l., 39100 Bozen (IT)
(72) Erfinder: Gallmetzer, Dietrich, 39018 Terlan (IT)
(74) Vertreter: Tergau & Walkenhorst Patentanwälte PartGmbB

(57) **Zusammenfassung**

Eine medizinische Spritze (1) mit einem das Spritzengehäuse bildenden, zur Aufnahme eines medizinischen Wirkstoffs vorgesehenen Hohlkörper (6), an dessen distalem Ende (8) ein Verbindungselement (10) angeformt ist, an dem ein Nadelhalter (12) für eine zur Injektion des Wirkstoffs vorgesehene Hohlnadel (14) befestigbar ist, soll für einen möglichst gering gehaltenen Materialverlust hinsichtlich des auszubringenden Wirkstoffs ausgelegt sein. Dazu weist erfindungsgemäß das Verbindungselement (10) einen zur Verbindung mit dem Nadelhalter (12) vorgesehenen Innenkanal (24) auf, der einen sich zum distalen Ende (8) hin kontinuierlich aufweitenden Innenquerschnitt aufweist.

## Beschreibung

Die Erfindung betrifft eine medizinische Spritze, insbesondere mit Nadelschutz, mit einem das Spritzengehäuse bildenden, zur Aufnahme eines medizinischen Wirkstoffs vorgesehenen Hohlkörper.

Medizinische Spritzen sind in vielfältigen, insbesondere therapeutischen, Anwendungen im Einsatz. Üblicherweise umfassen sie einen über einen Betätigungsstößel im Innenraum eines Spritzenkörpers verschiebbaren Kolben.

Zur Vermeidung oder Verringerung von Kontaminations- oder Verletzungsrisiken nach dem Gebrauch von Medikamentenspritzen und insbesondere zur Vermeidung des mehrfachen Gebrauchs von Spritzennadeln durch verschiedene Nutzer finden Spritzen mit so genannten Einzieh- oder Retraktionssystemen für die Spritzennadel zunehmend Verwendung. Bei solchen insbesondere medizinischen Spritzen, auch als "Spritze mit (passivem) Nadelschutz" bezeichnet, wird die Spritzennadel nach Abgabe des in der Spritze vorgehaltenen Wirkstoffs in den Spritzenkörper eingezogen und von diesem vollständig umschlossen. Ein Zugang zur Spritze und damit ein Verletzungsrisiko, oder auch das Risiko mehrfachen Gebrauchs derselben Nadel, kann damit weitgehend ausgeschlossen werden.

Derartige Spritzen mit passivem Nadelschutz sind beispielsweise aus der EP 1 284 769 B1, der EP 0 720 491 B1, der EP 0 680 347 B1 oder der EP 1 764 127 B1 bekannt.

Üblicherweise ist die Nadel einer medizinischen Spritze in einem Nadelhalter gehalten, der geeignet mit dem Spritzengehäuse verbunden, insbesondere auf dieses aufgeschraubt oder aufgeclipt, wird. Ganz allgemein bei medizinischen Spritzen, und somit auch bei Spritzen mit Nadelschutz der genannten Art, besteht das Bedürfnis, beim Gebrauch der Spritze den darin enthaltenen Wirkstoff möglichst vollständig abzugeben und den Verbleib nicht genutzter Restmengen des Wirkstoffs innerhalb der Spritze möglichst weitgehend zu minimieren.

Der Erfindung liegt nunmehr die Aufgabe zugrunde, eine Spritze mit Nadelschutz der oben genannten Art anzugeben, bei der auch bei nachträglich angebrachtem Nadelhalter diesem Bedürfnis Rechnung getragen ist.

Diese Aufgabe wird erfindungsgemäß gelöst, indem das Verbindungselement einen zur Verbindung mit dem Nadelhalter vorgesehenen Innenkanal aufweist, der einen sich zum distalen Ende hin kontinuierlich aufweitenden Innenquerschnitt aufweist.

Vorteilhafte Ausgestaltungen der Erfindung sind Gegenstand der Unteransprüche und/oder der nachfolgenden Figurenbeschreibung entnehmbar.

Die Erfindung geht von der Überlegung aus, dass gerade bei mehrkomponentigen Systemen, bei denen der Nadelhalter zunächst als separates Bauteil hergestellt und erst anschließend am Spritzengehäuse montiert wird, im Bereich der Verbindung zwischen Nadelhalter und Spritzengehäuse Undichtigkeiten und dadurch bedingt Leckagen auftreten könnten. Um dem entgegenzuwirken und auch bei dieser Bauweise eine ganz besonders hohe Dichtigkeit des Systems und damit die Vermeidung unbeabsichtigter, leckagebedingter Abgaben von Wirkstoff zu ermöglichen, sollte gerade der Verbindungsbereich für eine besonders hohe Dichtigkeit ausgelegt sein. Um dies zu ermöglichen, ist nunmehr die Nutzung einer konischen Innenverbindung für die wirkstoffseitige Verbindung von Nadelhalter und Spritzengehäuse vorgesehen. Dazu ist der hierfür vorgesehene Innenkanal im Verbindungsbereich des Spritzengehäuses mit sich aufweitendem Querschnitt in der Art eines Konus ausgeführt. Damit kann das korrespondierende Bauteil am Nadelhalter in diesen Konus eingesteckt werden, so dass gerade bei ausreichender Anpresskraft die erwünschte Dichtigkeit der Verbindung sichergestellt werden kann.

Vorteilhafterweise ist der Innenkanal dabei in der Art eines sich zum distalen Ende hin aufweitenden Konus mit einem Konuswinkel von etwa 2° ausgestaltet.

In vorteilhafter Weiterbildung oder in als eigenständig erfinderisch angesehener Ausführung ist die medizinische Spritze mit einem RFID-Chip, beispielsweise zum Zweck der Errichtung einer digitalisierten Logistik, eines automatisierbaren Transportsystems für Spritzen, zur Qualitäts- oder Produktkontrolle und/oder für anderweitige Erfassungs- oder Überwachungszwecke, ausgerüstet. Dieser weist vorteilhafterweise eine individualisierte, maschinenauslesbare Codierung auf. Vorzugsweise ist der RFID-Chip an einer am proximalen Ende des das Spritzengehäuse bildenden Hohlkörpers angeformten Griffplatte angeordnet.

In weiterer alternativer oder zusätzlicher vorteilhafter Weiterbildung oder auch in als eigenständig erfinderisch angesehener Ausführung umfasst die medizinische Spritze einen Betätigungsstößel, an dessen Schaft eine Anzahl von Anschlag- und Rastelementen, vorzugsweise ausgeführt als um den Schaft umlaufende Stellringe, angeordnet ist.

In ganz besonders bevorzugter Ausgestaltung ist der Spritzenkörper als Kunststoffteil, vorzugsweise aus Cyclo-Olefin-Polymer (COP) oder aus Cyclo-Olefin-Copolymer (COC), mit oder ohne Barriereschicht, gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist. Damit wird dieser Werkstoff erfindungsgemäß als sogar für die Primärverpackung auch anspruchsvoller Medikamente, insbesondere für sensible biotechnologisch hergestellte Wirkstoffe, geeignet angesehen und verwendet. Zudem ist dieser Werkstoff für eine Fertigung im Spritzgießverfahren und somit für besonders präzise Dimensionierung geeignet.

Ein Ausführungsbeispiel der Erfindung wird anhand einer Zeichnung näher erläutert. Darin zeigen:
- Fig. 1: eine medizinische Spritze im Längsschnitt in zwei Schnittebenen,
- Fig. 2: einen das Spritzengehäuse der Spritze nach Fig. 1 bildenden Hohlkörper im Längsschnitt und in seitlicher Ansicht,
- Fig. 3: einen Nadelhalter der Spritze nach Fig. 1 im Längsschnitt,
- Fig. 4: einen Betätigungsstößel der Spritze nach Fig. 1 in seitlicher und in perspektivischer Ansicht,
- Fig. 5: den Betätigungsstößel nach Fig. 4 mit angebrachtem Stopfen,
- Fig. 6: eine Schutzkappe der Spritze gem. Fig. 1 in perspektivischer Ansicht und im Längsschnitt,
- Fig. 7 ,8: die Spritze gem. Fig. 1 in perspektivischer Ansicht mit bzw. ohne Schutzkappe,
- Fig. 9: die Spritze gem. Fig. 1 in einer Betätigungsposition im Längsschnitt,
- Fig. 10: den Hohlkörper gem. Fig. 2 in perspektivischer Ansicht,
- Fig. 11,12: die Spritze gem. Fig. 1 in unterschiedlichen Betätigungspositionen im Längsschnitt,
- Fig. 13, 14: den Hohlkörper gem. Fig. 2 jeweils in perspektivischer Ansicht
- Fig. 15: eine mit RFID-Chip versehene Standard-Spritze,
- Fig. 16, 17: jeweils in Explosionsdarstellung eine medizinische Spritze mit passivem Retraktionsmechanismus, und
- Fig. 18: einen Hohlkörper der Spritze nach Fig. 16, 17.

Gleiche Teile sind in allen Figuren mit denselben Bezugszeichen versehen. Die Ausführungsbeispiele weisen eine Mehrzahl von als eigenständig erfinderisch angesehenen Merkmalen oder Merkmalsgruppen auf. Diese können erfindungsgemäß jeweils einzeln und unabhängig voneinander oder auch in beliebiger Kombination miteinander vorgesehen sein.

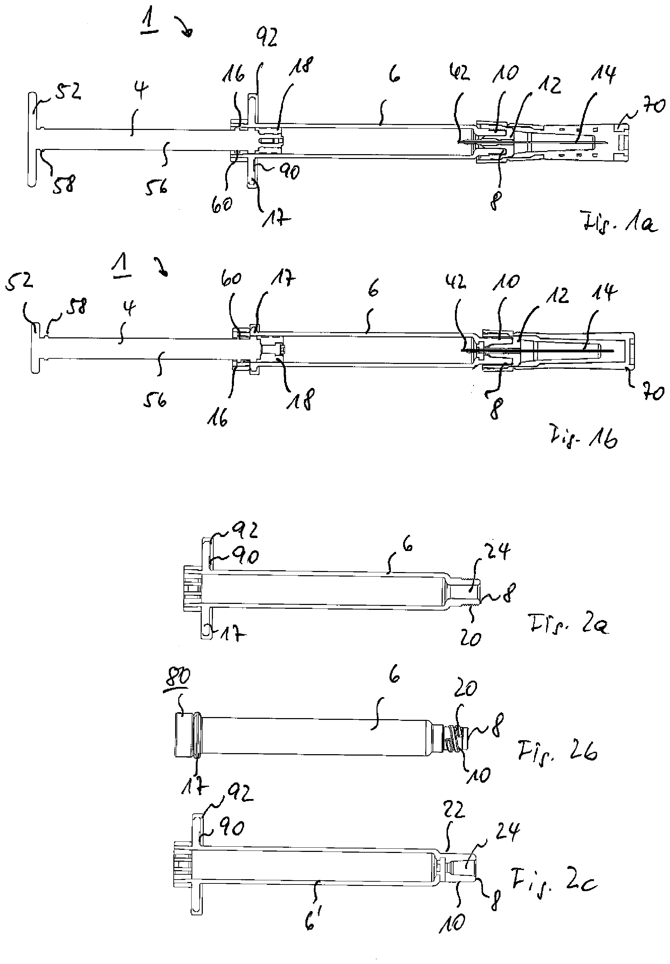

Die medizinische Spritze 1 mit Nadelretraktion, die in ihrer Gesamtheit in Fig. 1 im Längsschnitt gezeigt ist, umfasst einen Betätigungsstößel 4 und einen entsprechend einer herkömmlichen Bauweise zylindrisch oder rohrförmig ausgeführten, das Spritzengehäuse bildenden Hohlkörper 6, der zur Aufnahme des medizinischen Wirkstoffs vorgesehen ist. Am vorderen oder distalen Ende 8 des Hohlkörpers 6 ist ein Verbindungselement 10 angeformt, an dem ein Nadelhalter 12 für eine zur Injektion des Wirkstoffs vorgesehene Hohlnadel 14 befestigt werden kann. Das hintere oder proximale Ende 16 des das Spritzengehäuse bildenden Hohlkörpers 6, an dem in an sich bekannter Bauweise eine Gegen- oder Griffplatte 17 angeformt ist, ist hingegen von einem in seinen Außenabmessungen passgenau an die Innenkontur des Hohlkörpers 6 angepassten, innerhalb des Hohlkörpers 6 verschiebbaren Kolben oder Stopfen 18 verschließbar. Die medizinische Spritze 1 könnte grundsätzlich als mit dem Wirkstoff vorbefüllte Spritze 1 ausgelegt sein oder alternativ auch derart eingesetzt werden, dass die Spritze 1 leer einsatzfähig gemacht und durch Aufziehen mit dem Wirkstoff befüllt wird. Beide Einsatzarten werden hinsichtlich der nachfolgend beschriebenen detaillierten Ausgestaltungsmekmale als gleichwertig und alternativ zueinander einsetzbar angesehen.

Im Hinblick auf eine gute und zuverlässige Einsetzbarkeit auch in modernen Therapien und die dabei ggf. zur Anwendung kommenden empfindlichen, hochpreisigen und unter Umständen bei unsachgemäßer Anwendung auch toxischen Wirkstoffe ist die Spritze und insbesondere der das Spritzengehäuse bildendende Hohlkörper 6 für eine besonders hohe Dichtigkeit ausgelegt, so dass gerade bei der Verwendung als vorbefüllte Spritze auch eine gewisse Lagerfähigkeit des darin vorgehaltenen Wirkstoffs erreichbar ist. Dazu ist einerseits in als eigenständig erfinderisch angesehener Weise das Material für das Spritzengehäuse bzw. den dieses bildenden Hohlkörper 6 geeignet gewählt, so dass auch hohen Ansprüchen an die zuverlässige vorübergehende Lagerung des medizinischen Wirkstoffs einhergehend mit einer besonders hohen Sicherheit im Umgang mit den Komponenten Rechnung getragen wird. Der als zylindrischer Hohlkörper ausgeführte Spritzenkörper 6 kann dabei aus PP oder aus Cyclo-Olefin-Copolymer (COC), mit oder ohne Barriereschicht, ausgeführt sein, ist aber in als erfinderisch angesehener Ausgestaltung besonders bevorzugt aus dem Hochleistungskunststoff Cyclo-Olefin-Polymer (COP) gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist. Der Spritzenkörper 6 ist vorzugsweise im Spritzgießverfahren gefertigt, wobei unter anderem die Formgebung derart erfolgt, dass mögliche Totvolumina im Inneren besonders gering gehalten sind.

Andererseits ist der Hohlkörper 6 aber auch durch die geometrische Gestaltung und Formgebung seiner Komponenten für die beabsichtigte hohe Dichtigkeit und damit die Absicherung gegen unbeabsichtigten Materialverlust geeignet ausgelegt. Dem wird im Bereich des distalen Endes 8 insbesondere durch die als eigenständig erfinderisch angesehene Ausgestaltung der Geometrie des Verbindungselements 10 und der von diesem gemeinsam mit dem Nadelhalter 12 gebildeten Komponentenverbindung Rechnung getragen.

Der Nadelhalter 12 kann dabei auf den das Spritzengehäuse bildenden Hohlkörper 6 bzw. den Spritzenkonus aufsteckbar oder alternativ auch mittels eines Gewindes, beispielsweise eines Luer-Gewindes, aufschraubbar ausgeführt sein. Beide Varianten werden vorliegend als gleichwertige Alternativen angesehen, wobei die diesbezügliche Ausführung der Verbindung als austauschbar und im Hinblick auf sonstige Randbedingungen, beispielsweise der beabsichtigten späteren Handhabung, frei auswählbar angesehen wird. Im Ausführungsbeispiel gemäß Figur 2 ist dementsprechend in Fig. 2a im Längsschnitt und in Fig. 2b in seitlicher Ansicht ein das Spritzengehäuse bildender Hohlkörper 6 gezeigt, dessen Verbindungselement 10 mit einem zur Herstellung einer Schraubverbindung vorgesehenen Außengewinde 20 versehen ist. Dieses ist, wie in Fig. 2b gezeigt, bevorzugt als Luer-Gewinde ausgestaltet, kann aber auch als Feingewinde ausgeführt sein, wie dies in Fig. 2a gezeigt ist. Demgegenüber ist in Fig. 2c im Längsschnitt ein das Spritzengehäuse bildender Hohlkörper 6' gezeigt, dessen Verbindungselement 10 zur Herstellung einer Clip-, Aufsteck- oder Aufprellverbindung mit einer eine Rastkante 22 aufweisenden Haltekontur, beispielsweise in Form eines Außensechskants, versehen ist.

Beiden Varianten ist die als eigenständig erfinderisch angesehene Ausgestaltung gemeinsam, dass das Verbindungselement 10 jeweils einen zur Verbindung mit dem Nadelhalter 12 vorgesehenen Innenkanal 24 aufweist, der einen sich nach außen, also zum distalen Ende 8 hin, kontinuierlich aufweitenden Innenquerschnitt aufweist. Im Ausführungsbeispiel ist der Innenkanal 24 dabei jeweils in der Art eines sich zum freien Ende hin aufweitenden Konus mit zumindest annähernd kreisförmigem Durchschnitt ausgestaltet. Der Konuswinkel ist dabei im Hinblick auf die beabsichtigte, nachfolgend näher erläuterte Dichtwirkung geeignet gewählt; im Hinblick auf die bevorzugt gewählte Materialpaarung des Hohlkörpers 6 und des Nadelhalters 12 beträgt der Konuswinkel bevorzugt etwa 2°.

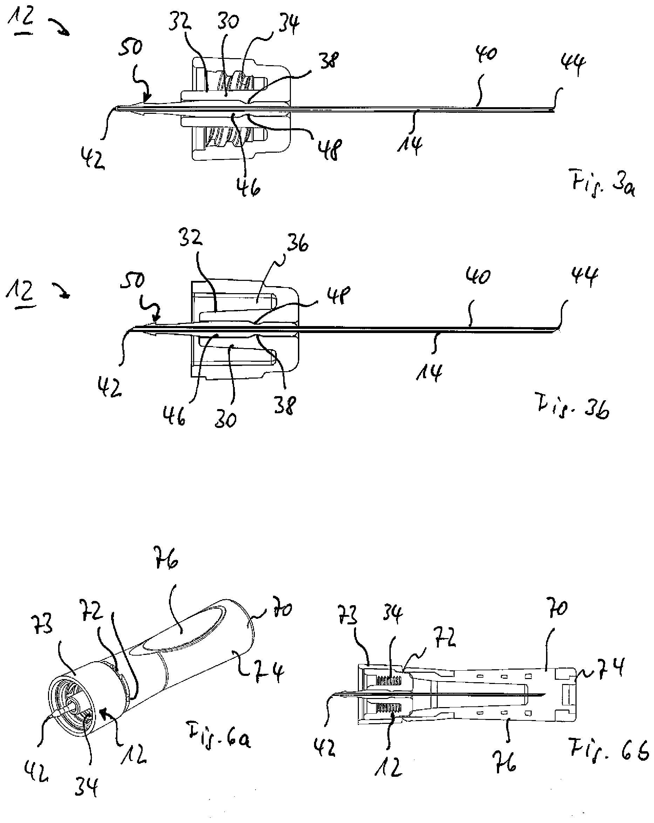

Der die Hohlnadel 14 tragende Nadelhalter 12 ist in Fig. 3 im Detail im Längsschnitt gezeigt, und zwar in Fig. 3a in einer für die Schraubverbindung und in Fig. 3b für die Clip- oder Steckverbindung vorgesehenen Variante. Er weist jeweils eine Lagermuffe 30 für die Hohlnadel 14 auf. Diese weist eine in ihrer Dimensionierung, also hinsichtlich ihrer Querschnittsfläche, ihres Durchmessers und ihres Konuswinkels, an den Innenkanal 24 des Verbindungselements 10 angepasste, in diesen einsteckbare konisch ausgeführte Kontaktfläche 32 auf. Der Nadelhalter 12 und/oder dessen Stopfen 30 kann ein- oder auch mehrkomponentig, insbesondere zweikomponentig, ausgebildet sein. Durch die genannte Ausgestaltung des Verbindungssystems kann unter gezielter Nutzung der konischen Ausgestaltung der Verbindungsflächen eine besonders hohe Dichtwirkung erzielt werden, mit der der Innenkanal 24 durch den Nadelhalter 12 verschlossen wird. Die eigentliche Fixierung am Verbindungselement 10 erfolgt dann bei der in Fig. 3a gezeigten Variante der Schraubverbindung mittels eines in den Nadelhalter 12 integrierten, an das Außengewinde 20 angepassten und somit ebenfalls als Luer-Gewinde oder Feingewinde ausgestalteten Innengewindes 34 und bei der in Fig. 3b gezeigten Variante der Clip- oder Steckverbindung mittels einer an die Haltekontur angepassten Innenkontur, im Ausführungsbeispiel einem Innensechskant 36.

Die Ausgestaltung der Hohlnadel 14 und ihre Fixierung in der Lagermuffe 30 entsprechen im Wesentlichen der Ausführung des in der EP 3 738 629 A1 beschriebenen Systems. Analog dazu ist auch vorliegend die Lagermuffe 30 mit einer umlaufenden Fixierlippe 38 versehen. Die Hohlnadel 14 umfasst ein Nadelrohr 40 aus Metall, das an seinen beiden Enden jeweils eine Nadelspitze 42, 44 bildet. Die Materialwahl für das Nadelrohr 40 ist dabei bevorzugt im Hinblick auf gängige Erfordernisse für medizinische Anwendungen geeignet erfolgt, wobei besonders bevorzugt ein auch für Standard-Nadeln verwendbares rostfreies Material, insbesondere Edelstahl, vorgesehen ist. In seinem mittleren Längenbereich ist das Nadelrohr 40 ummantelt ausgeführt und von einem Kunststoffmantel 46 umgeben oder umspritzt. Als Material für den Kunststoffmantel 46 ist dabei vorzugsweise ein Polyamid (PA12), ganz besonders bevorzugt das im Handel unter der Bezeichnung Vestamid Care ML 17 Erhältliche, vorgesehen. Der Kunststoffmantel 46 wird dabei in einer ganz besonders bevorzugten Ausgestaltung auf das Nadelrohr 40 aufgespritzt, nachdem dieses in der Art einer Oberflächenaktivierung einer Plasmavorbehandlung unterzogen wurde. Damit ist eine besonders gute Haftung des den Kunststoffmantel 46 bildenden Kunststoffs auf der Nadel 40 erreichbar.

Im Kunststoffmantel 46 ist eine Haltenut 48 eingeformt, die der vorübergehenden Fixierung der Nadel 14 in der Lagermuffe 30 des Nadelhalters 12 dient. Dabei greift die Fixierlippe 38 der Lagermuffe 30 bei montierter und in die Lagermuffe 30 ordnungsgemäß eingeschobener Nadel 14 in die Haltenut 48 ein und fixiert diese in Längsrichtung. Die Dimensionierungen der Haltenut 48 und der Fixierlippe 38 sind derart gewählt, dass unter Berücksichtigung der Verformbarkeit des Materials des Kunststoffmantels 46 die umspritzte Nadel 14 in die Lagermuffe 30 eingeschoben und dort eingeclipt oder eingerastet werden kann. Des Weiteren ist der Kunststoffmantel 46 in seinem der innenseitigen Nadelspitze 42 zugewandten Endbereich zur Ausbildung einer Rastkante 50 oder einer Anzahl von Rastelementen, beispielsweise Rasthaken oder dergleichen, ausgestaltet.

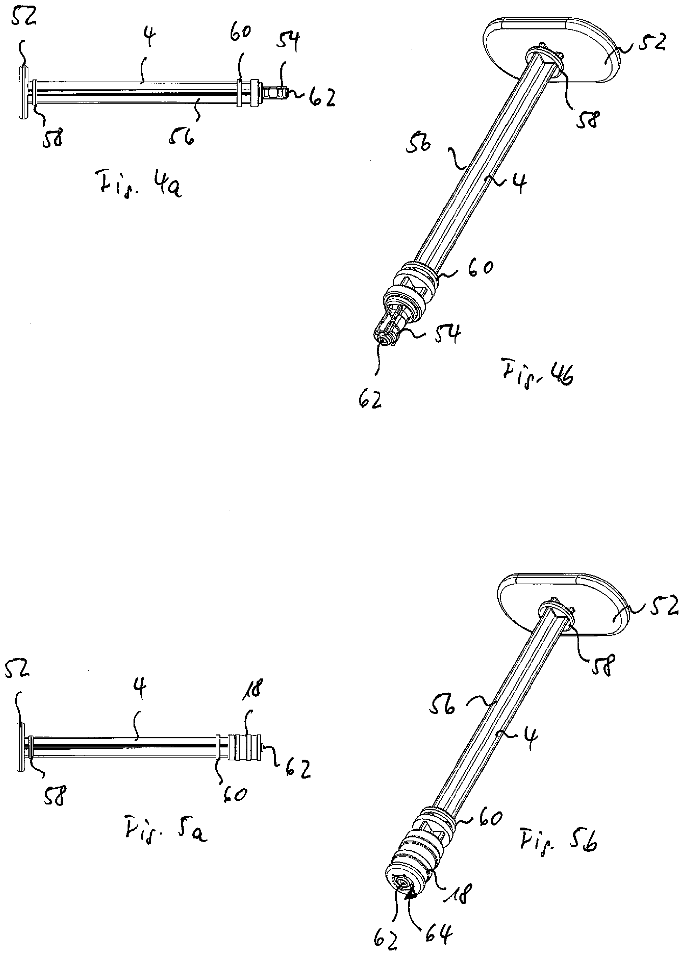

Der in Fig. 4 in Draufsicht und perspektivisch alleine und in Fig. 5 mit angebrachtem Stopfen 18 gezeigte Antriebs- oder Betätigungsstößel 4 umfasst einen einerseits endseitig mit einer Drückerplatte 52 versehenen und andererseits an seinem der Drückerplatte 52 gegenüberliegenden Ende mit einem zur Aufnahme der Hohlnadel 14 vorgesehenen Greifbügel 54 versehenen Schaft 56. In als eigenständig erfinderisch angesehener Ausgestaltung sind am Schaft 56 zudem eine Anzahl von Anschlag- und Rastelementen angeordnet, die im Ausführungsbeispiel als Stellringe 58, 60, nämlich einem proximal im der Drückerplatte 52 benachbarten Bereich des Schafts 56 angeordneten ersten Stellring 58 und einem distal im dem Greifbügel 54 benachbarten Bereich des Schafts 56 angeordneten zweiten Stellring 60, ausgeführt sind. Auslegung und Funktionsweise dieser Anschlag- und Rastelemente werden nachstehend noch näher erläutert.

Die medizinische Spritze 1 ist durch die genannten Komponenten und Bauteile mit einem in der Art eines Retraktionssystems ausgeführten Nadelschutz versehen. Damit wird bezweckt, dass nach der Benutzung der Spritze 1, also nach der Ausgabe des im das Spritzengehäuse bildenden Hohlkörper 6 vorgehaltenen Wirkstoffs über die Nadel 14, diese in das Spritzengehäuse derart hineingezogen wird, dass sie vom Spritzengehäuse vollständig umschlossen wird. Damit soll eine unbeabsichtigte Berührung der benutzten Nadel 14, beispielsweise durch Hilfs- oder Pflegepersonal, und somit das Verletzungs- und Kontaminationsrisiko besonders gering gehalten oder möglichst ganz ausgeschlossen werden. Die medizinische Spritze 1 ist dazu mit einem im Wesentlichen der Ausführung des in der EP 3 738 629 A1 beschriebenen Systems entsprechenden Retraktionssystem versehen.

Dazu ist der Greifbügel 54 mittig mit einem ein Aufnahmeloch für die Nadel 14 bildenden Nadellager 62 versehen, in das die innenseitige Nadelspitze 42 eingebracht und mittels der Rastkante 50 oder anderer geeigneter Rastmittel verrastet werden kann. Das Nadellager 62 ist dabei, analog der oben beschriebenen Lagermuffe 30, innenseitig mit einer zugeordneten umlaufenden Rastlippe versehen, die bei in das Nadellager 62 eingeschobener Nadelspitze 42 in die durch die Rastkante 50 gebildete Haltenut eingreift und die Nadel 14 somit in Längsrichtung fixiert. Die Dimensionierungen der Haltenut und der dieser zugeordneten Rastlippe im Nadellager 62 sind dabei derart gewählt, dass unter Berücksichtigung der Verformbarkeit des Materials des Kunststoffmantels 46 und/oder einer eventuellen Klebekraft durch die Materialpaarung des Materials des Kunststoffmantels 46 und des diesen umgebenden Materials des Nadellagers 62 die Halte- oder Losbrechkraft der solchermaßen eingerasteten Nadel 14 in Längsrichtung größer ist als die entsprechende Halte- oder Losbrechkraft der Fixierlippe 38 in der Lagermuffe 30, so dass bei einer Retraktionsbewegung des Stopfens 18 in das Innere des Hohlkörpers 6,6' die Nadel 14 zum Inneren des Hohlkörpers 6,6' mitgenommen wird. Der Greifbügel 54 ist dabei als Kunststoffteil ausgeführt und besteht im Ausführungsbeispiel im Hinblick auf die erforderlichen Haltekräfte und die bei bestimmungsgemäßem Gebrauch erwarteten mechanischen Belastungen, aber auch im Hinblick auf zulassungsbedingte Erfordernisse, aus dem unter der Bezeichnung "Bormed^{™}" (HD810MO), ISO 10993 Information (Biocompatibility)) erhältlichen Polypropylen oder alternativ aus anderen, pharmatauglichen Polyolefinen mit entsprechenden Eignungen.

Ebenfalls analog zum in der EP 3 738 629 A1 beschriebenen System wird durch die Ausgestaltung des Greifbügels 54 in Kombination mit dem ihn umgebenden Stopfen 18 innerhalb des Stopfens 18 ein Freiraum 64 geschaffen, der in der Endphase der Ausbringung des Wirkstoffs, bei der die Nadelspitze 42 schon in das Nadellager 62 eingedrungen und somit für den Wirkstoff nicht mehr ohne weiteres zugänglich ist, in der Art eines Bypasses das Einströmen des Wirkstoffs über die Nadelspitze 42 in das Nadelrohr 40 ermöglicht. Der Zustrom kann dabei beidseitig des Greifbügels 54 in den Freiraum 64 hinein erfolgen. Der somit als Kolbenmantel fungierende Stopfen 18 besteht dabei vorteilhafterweise unter Berücksichtigung zulassungsbedingter Erfordernisse aus herkömmlichem Gummi, besonders bevorzugt aus dem von der Firma Kreiburg unter der Bezeichnung TM4RST (MC/RS Series) erhältlichen Material. Der Stopfen 18 ist dabei derart geformt, dass er bei eingebrachtem Greifbügel 54 beidseitig des Greifbügels 54 Zuströmkanäle für den Wirkstoff freilässt, so dass im Sinne einer Vermeidung oder Minimierung des Totvolumens der Bypass für den angestrebten Nullvolumen-Ausstoß gebildet wird. Zur Vermeidung von Verletzungen oder dergleichen bei der Handhabung der medizinischen Spritze 1 ist diese weiterhin mit einer abnehmbaren Schutzkappe 70 für die Nadel 14 versehen, die vor dem Einsatz der Spritze 1 entfernt wird. Diese in Fig. 6 dargestellte Schutzkappe 70 ist in ganz besonders bevorzugter Ausgestaltung als so genannter Originalitätsverschluss in der Art eines Einwegverschlusses ausgestaltet sein. Diese Ausgestaltung als Einweg- oder Originalitätsverschluss erlaubt eine problemlose und zuverlässige Identifikation, ob die Spritze 1 bereits zum Flüssigkeitstransfer verwendet wurde oder nicht, und erleichtert somit die Zuordnung, ob die Spritze 1 beispielsweise noch das Erfordernis der Sterilität bzw. der Einhaltung der Sterilbarriere erfüllt oder nicht, oder ob - bei Verwendung als vorbefüllte Spritze - der darin vorgehaltene Wirkstoffvorrat bereits "angebrochen" wurde oder nicht. Wie der Darstellung in Fig. 6a in perspektivischer Ansicht und in Fig. 6b im Längsschnitt entnehmbar ist, ist die Schutzkappe 70 dabei über eine Anzahl von Verbindungsstegen 72 stoffschlüssig mit einem Abreiß- oder Sicherungsring 73 verbunden, d. h. beispielsweise angeformt oder angegossen. Der Abreiß- oder Sicherungsring 73 kann dabei rastend auf den Nadelhalter 12 aufgeclipt werden .Zum Entfernen der Schutzkappe 70 muss diese somit vom Abreiß- oder Sicherungsring 73 abgebrochen werden, wobei die Verbindungsstege 72 unreparierbar durchtrennt werden. Die den Originalitätsverschluss bildende Schutzkappe 70 ist dabei im Ausführungsbeispiel zweikomponentig ausgeführt und umfasst das eigentliche Kappengehäuse 74 (bevorzugt gebildet aus PP) sowie ein Bedienfeld 76 (bevorzugt aus TPE).

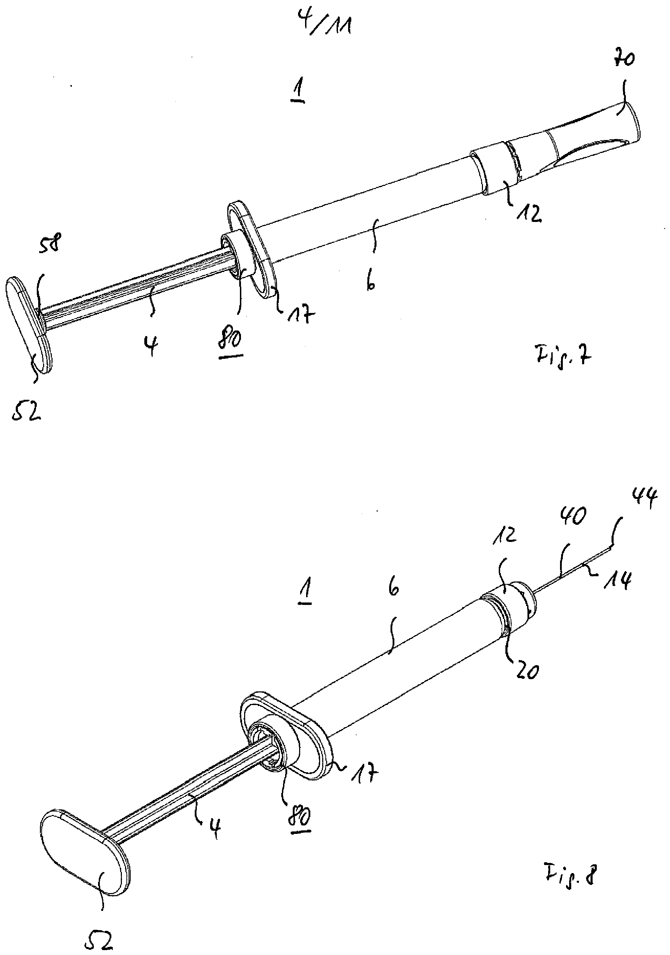

Die medizinische Spritze 1 in ihrer Gesamtheit, also mit Betätigungsstößel 4, dem das Spritzengehäuse bildenden Hohlkörper 6,6' und angebrachtem Originalitätsverschluss, vor ihrem erstmaligen Gebrauch ist in Fig. 7 in perspektivischer Ansicht und, wie bereits erläutert, in Fig. 1 im Längsschnitt gezeigt. Beim Einsatz und der Handhabung der genannten Komponenten ist grundsätzlich der folgende Ablauf vorgesehen:
Als erster Schritt muss die Schutzkappe 70 durch Abbrechen entfernt werden. Anschließend ist die Spritze 1, wie in Fig. 8 gezeigt, einsatzbereit. Die Spritze 1 könnte grundsätzlich mit vorbefülltem Hohlkörper 6, also als vorbefüllte Spritze, eingesetzt werden. In diesem Fall ist das System im in Fig. 8 gezeigten Zustand bereits zur Ausgabe des Wirkstoffs bereit.

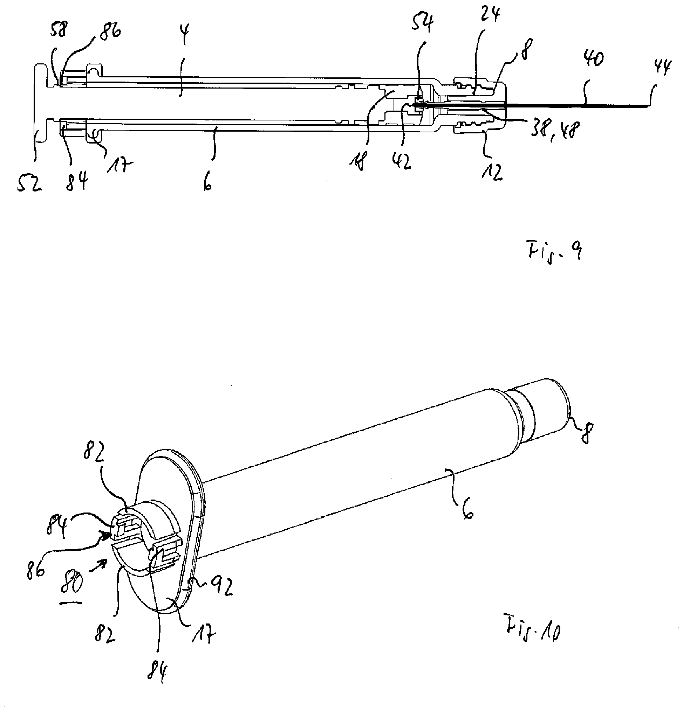

Im Ausführungsbeispiel ist jedoch vorgesehen, dass die Spritze 1 leer einsatzfähig gemacht und durch Aufziehen mit dem Wirkstoff befüllt wird. Dazu wird zunächst der Betätigungsstößel 4 vom Bediener gedrückt, so dass sich der Stopfen 18 innerhalb des Hohlkörpers 6 auf dessen distales Ende 8 hin zubewegt. Sobald dabei eine vorgegebene Endposition erreicht wird, bei der der Stopfen 18 noch ausreichend weit von der Nadelspitze 42 entfernt ist, so dass die Rastkante 50 nicht in das Nadellager 62 im Greifbügel 54 eindringen kann, wird diese Bewegung durch einen durch Anschlag gestoppt. Die Spritze 1 in dieser Position ist in Fig. 9 im Längsschnitt gezeigt. Der Anschlag wird dabei in als eigenständig erfinderisch angesehener Ausgestaltung durch das Zusammenspiel des proximalen Stellrings 58 mit einer außenseitig an der Griffplatte 17 des Hohlkörpers 6 angeformten Rastaufnahme 80 gebildet.

Die Rastaufnahme 80, deren genaue Ausführung gemäß Ausführungsbeispiel in der perspektivischen Ansicht auf die Griffplatte 17 des Hohlkörpers 6 gemäß Fig. 10 gut erkennbar ist, umfasst eine Anzahl von in ihrer Gesamtheit eine Führungskontur bildenden, an die Griffplatte 17 angeformten Ringsegmenten 82, die unter Bildung von Lücken eine nicht vollständig durchgängige umlaufende Kontur bilden. In den Lücken sind dabei jeweils nach außen biegbare Rasthaken 84 angeordnet, die auf ihrer Oberseite 86 jeweils eine Anschlagfläche für den proximalen Stellring 58 bilden.

Beim Einschieben des Stopfens 18 in das Innere des Hohlkörpers 6 ist dieses Anschlagen des proximalen Stellrings 58 an die Oberseite 86 der Rasthaken 84 für den Benutzer oder Bediener tastbar, so dass er die Einschubbewegung in der in Fig. 9 gezeigten Position stoppen kann. Die Positionierung des Stellrings 58 auf dem Schaft 56 ist dabei in als erfinderisch angesehener Ausgestaltung derart gewählt, dass der Stopfen 18 noch ausreichend weit von der Nadelspitze 42 entfernt ist, so dass die Rastkante 50 nicht in das Nadellager 62 im Greifbügel 54 eindringen kann. Damit ist sichergestellt, dass ein unbeabsichtigtes "zu frühes" Auslösen des Retraktionsmechanismus vermieden werden kann. In dieser Position kann nunmehr die frei liegende distale Nadelspitze 44 der Nadel 14 in ein externes Reservoir des Wirkstoffs, beispielsweise eine Phiole oder einen Wirkstoffbehälter, eingebracht werden. Anschließend wird der Stopfen 18 mittels des Betätigungsstößels 4 im Hohlkörper 6 zurückgezogen. Dabei wird der Wirkstoff über die Nadel 14 angesaugt und der Innenraum des Hohlkörpers 6 somit befüllt.

Nachdem die Spritze 1 auf eine der genannten Weisen einsatzfertig gemacht wurde und dementsprechend im in den Figuren 1 und 8 gezeigten Zustand vorliegt, wird die Nadel 14 zur Verabreichung des medizinischen Wirkstoffs geeignet am Patienten positioniert, so dass sie an geeigneter Stelle die Haut des Patienten durchstößt. Die Haltekraft der Nadel 14 in der Lagermuffe 30 ist dabei, insbesondere wie oben beschrieben durch die geeignete Dimensionierung der Komponenten und/oder die Wahl der Materialpaarung, derart vorgegeben, dass die Nadel 14 sicher in ihrer Position in der Lagermuffe 30 verbleibt, wenn der Bediener durch Handhabung am Hohlkörper 6 die Nadel 14 durch die Haut des Patienten hindurchsticht.

Anschließend wird der Betätigungsstößel 4 vom Bediener gedrückt, so dass sich der Kolben oder Stopfen 18 innerhalb des Hohlkörpers 6 auf dessen distales Ende 8 hin bewegt und dabei den medizinischen Wirkstoff der Nadel 14 zuführt und über diese ausbringt. Kurz vor vollständiger Ausbringung des Wirkstoffs, also kurz vor vollständiger Entleerung des Innenraums des Hohlkörpers 6, erreicht der Stopfen 18 in der Nähe des distalen Endes 8 des Hohlkörpers 6 die in den Innenraum ragende Nadelspitze 42 der Nadel 14, so dass diese bei weiterer Bewegung in das dafür vorgesehene Aufnahmeloch im Nadellager 62 des Greifbügels 54 eindringt. Das System nimmt somit erneut wieder die bereits in Fig. 9 gezeigte Position ein, in der der proximale Stellring 58 an der Oberfläche 86 der Rasthaken 84 anschlägt. Erneut nimmt der Bediener dies haptisch wahr.

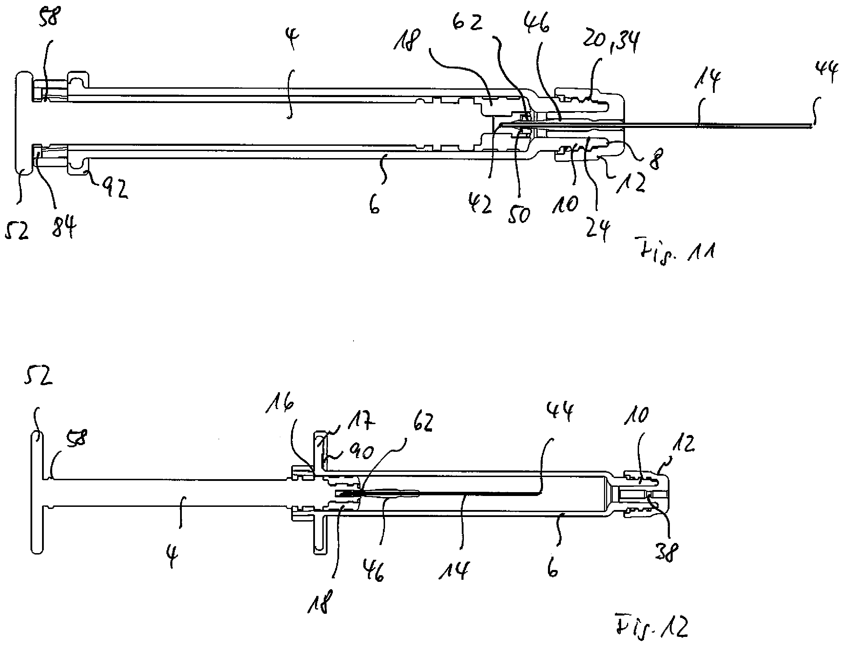

Anders als zuvor ist nunmehr aber die vollständige Ausbringung des Wirkstoffs vorgesehen. Dazu wendet der Bediener eine etwas erhöhte Kraft zum weiteren Vorschieben des Stopfens 18 im Hohlkörper 6 auf, wobei die Rasthaken 84 aufgrund geeigneter Formgebung und Konturierung an ihrer Oberfläche nach außen hin ausweichen, so dass der Stellring 58 den genannten Anschlag überwindet. Anschließend erreicht der Stopfen 18, wie dies im Längsschnitt in Fig. 11 gezeigt ist, dann seine Endposition (oder auch die "Nullstellung") unmittelbar am distalen Ende 8 des Hohlkörpers 6. Dabei umschließt das Nadellager 62 den in das Aufnahmeloch hineinragenden endseitigen Teil der Hohlnadel 14, wobei die innenseitig im Nadellager 62 umlaufende Rastlippe in die durch die Rastkante 50 im Kunststoffmantel 46 der Nadelumspritzung gebildete Haltenut eingreift und die Nadel 14 somit in Längsrichtung fixiert. Diese Einbringung des entsprechenden Teilbereichs der Hohlnadel 14 in das Nadellager 62 und die dadurch erreichte Verbindung der Nadel 14 mit dem Greifbügel 54 wird vorliegend auch als "connecting" bezeichnet.

Als eigenständig erfinderisch wird in diesem Zusammenhang die Kombination des im Stopfen 18 durch das Zusammenwirken mit der Ausgestaltung des Greifbügels 54 gebildeten Freiraums, also des genannten Bypasses, mit dem sich in der Art eines Konus nach außen hin aufweitenden Innenkanal 24 im Nadelhalter 12 angesehen. Durch diese Kombination kann auch in der Endphase bei der Verabreichung des Wirkstoffs unter Minimierung eines eventuellen Totvolumens eine kontinuierliche, gleichmäßige Ausbringung des Wirkstoffs ermöglicht werden, so dass selbst für die Restmengen des zu verabreichenden Wirkstoffs eine kontinuierliche Injektion und hoch genaue Dosierung des Wirkstoffs gewährleistet werden kann, ohne dass die zur weiteren Bewegung des Betätigungsstößels 4 erforderliche Vorschubkraft verändert werden müsste.

Nach der Verabreichung des Wirkstoffs und insbesondere nach vollständiger Entleerung des Hohlkörpers 6 wird vom Bediener die Drückerplatte 52 und mit dieser auch der Betätigungsstößel 4 insgesamt wieder zurückgezogen. Damit wird auch der endseitig am Schaft 56 angeordnete Stopfen 18 mitgenommen und innerhalb des Hohlkörpers 6 vom distalen Ende 8 weg zum proximalen Ende 16 hin gezogen. Dabei nimmt er seinerseits die im Greifbügel 54 eingefasste und fixierte Nadel 14 mit und zieht diese in den Hohlkörper 6 hinein, so dass diese im Endzustand vollständig innerhalb des Hohlkörpers 6 positioniert ist. Diese Position ist im Längsschnitt in Fig. 12 gezeigt. In dieser Position ist die Nadel 14 sicher innerhalb des Hohlkörpers 6 aufbewahrt und vollständig von diesem umschlossen, so dass keine Gefahr mehr von der Nadel 14 für Personal oder Bediener, beispielsweise durch unbeabsichtigtes Stechen, ausgeht. Zur Fixierung dieser Position ist der zweite, distale Stellring 60 vorgesehen, der geeignet mit einem am Hohlkörper 6 angeordneten Rastelement verrastet wird. Damit ist der flansch-seitige Stellring 60 am Hohlkörper 6 sicher verankert, und die im Hohlkörper 6 befindliche Nadel 14 kann weder nach vorne noch nach hinten weitergezogen, geschoben oder gestoßen werden.

Bei der beschriebenen Funktionsweise kommt insbesondere zum Tragen, dass durch die geeignete Dimensionierung der Komponenten und/oder eine eventuellen Klebekraft durch die Materialpaarung des Materials des Kunststoffmantels 46 und des diesen umgebenden Materials der Lagermuffe 30 die Halte- oder Losbrechkraft der solchermaßen eingerasteten Nadel 14 in Längsrichtung zwar einerseits ausreichend groß ist, so dass die Nadel 14 entsprechend der oben beschriebenen Vorgehensweise in die Haut des Patienten eingestochen werden kann, andererseits aber auch ausreichend klein ist, so dass die beschriebene Retraktionsbewegung der Nadel 14 zum Inneren des Hohlkörpers 6 hin durchgeführt werden kann.

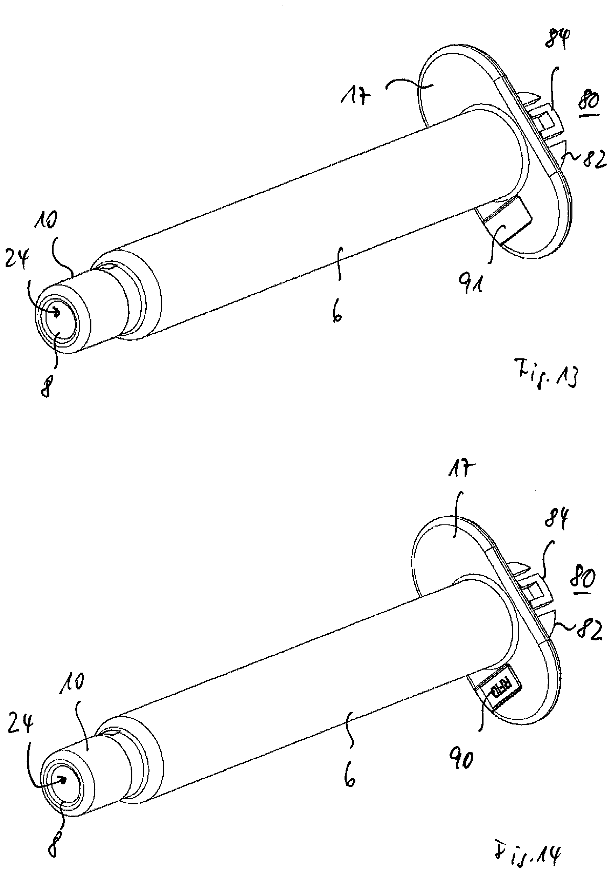

In weiterer als eigenständig erfinderisch angesehener Ausgestaltung ist die medizinische Spritze 1, insbesondere zum Zweck der Errichtung einer digitalisierten Logistik, eines automatisierbaren Transportsystems für Spritzen, zur Qualitäts- oder Produktkontrolle und/oder für anderweitige Erfassungs- oder Überwachungszwecke, mit einem RFID-Chip 90 ausgerüstet, der beispielsweise eine individualisierte, maschinenauslesbare Codierung aufweisen kann. Wie der Darstellung der Spritze 1 in Fig. 1 und besonders deutlich der perspektivischen Darstellung des Hohlkörpers 6 in Fig. 13, 14 entnehmbar ist, ist in dieser erfinderischen Ausgestaltung der RFID-Chip 90 an der an den Hohlkörper 6 angeformten Griffplatte 17 positioniert. Diese ist dazu mit einer Ausformung 91 zur Aufnahme des RFID-Chips 90 und zusätzlich mit einer aufgeclipten Fingerauflage 92 versehen, die einerseits den RFID-Chip 90 vollständig abdeckt und somit schützt und andererseits für den Benutzer eine angenehme Haptik bei der Bedienung erzeugt. Dazu ist die Fingerauflage 92 aus PP gefertigt.

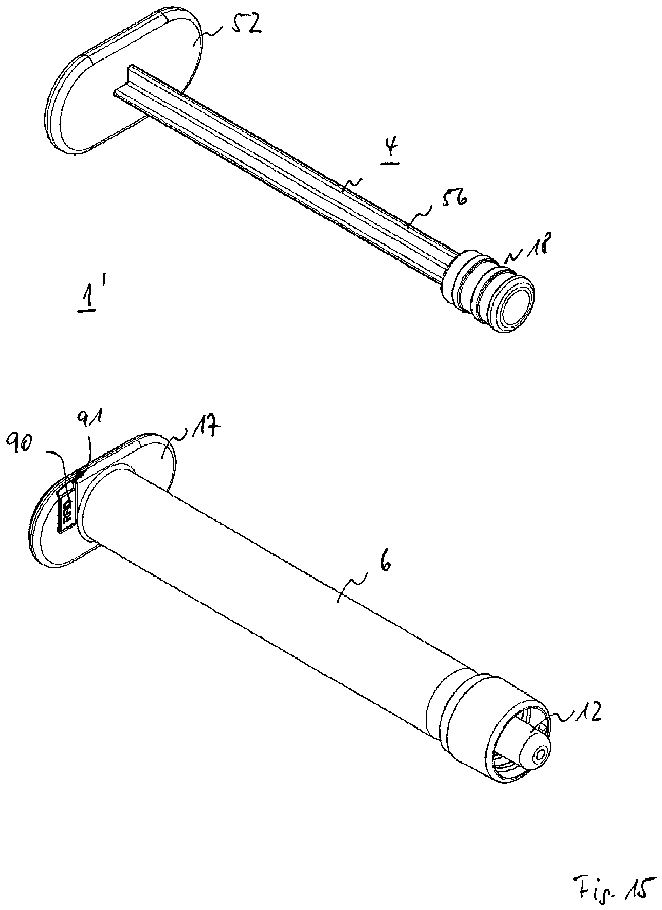

Eine derartige Ausrüstung einer medizinischen Spritze 1' mit einem RFID-Chip 90 im Bereich ihrer Griffplatte 17 wird als eigenständig erfinderisch angesehen. Dementsprechend ist in Fig. 15 eine an sich als Standard-Spritze mit herkömmlichem Betätigungsstößel 4 und herkömmlichem, das Spritzengehäuse bildendem Hohlkörper 6 ausgeführte medizinische Spritze 1' gezeigt, die an ihrer Griffplatte 17 mit einem solchen RFID-Chip 90 ausgerüstet ist. Auch diese Variante der medizinischen Spritze 1' kann selbstverständlich mit der als Originalitätsverschluss ausgeführten Schutzkappe 70 ausgerüstet sein.

Die vorstehend beschriebene medizinische Spritze 1 ist wie erläutert mit einem Retraktionssystem ausgerüstet, bei dem der Bediener nach vollständigem Ausbringen des Wirkstoffs die Nadel 14 vollständig in den das Spritzengehäuse bildenden Hohlkörper 6 zurückziehen kann. Dazu muss in dieser Ausführungsform nach dem erwähnten "Connecting" der Betätigungsstößel 4 mitsamt dem endseitig angeordneten Stopfen 18 und dem von diesem umschlossenen Nadellager 62, in dem nach dem "Connecting" die Nadel 14 fixiert ist, manuell oder in der Art einer aktiven Handlung durch den Bediener zurückgezogen werden, bis der distale Stellring 60 in der beschriebenen Weise mit dem Hohlkörper 6 verrastet wird. Diese Art des Retraktionssystems wird daher auch als "aktive Retraktion" bezeichnet.

In alternativer Ausführungsform kann eine medizinische Spritze 1 " auch mit einem für eine "passive Retraktion" geeigneten, "passiven" Retraktionssystem ausgerüstet sein, wie es seiner grundsätzlichen Funktionsweise nach beispielsweise aus der EP 3 738 628 A1 bekannt ist. Die Kombination eines solchen passiven Retraktionssystems mit einem oder mehreren der vorstehend beschriebenen Merkmale oder Merkmalsgruppen, insbesondere mit der Ausgestaltung der Verbindung mit dem Nadelhalter 12, mit der Ausrüstung mit RFID-Chip 90, mit der Ausrüstung des Betätigungsstößels 4 mit den Stellringen 58, 60 als Rast- oder Anschlagselemente und/oder mit der Ausrüstung mit einer als Originalitätsverschluss ausgeführten Schutzkappe 70, wird als eigenständig erfinderisch angesehen.

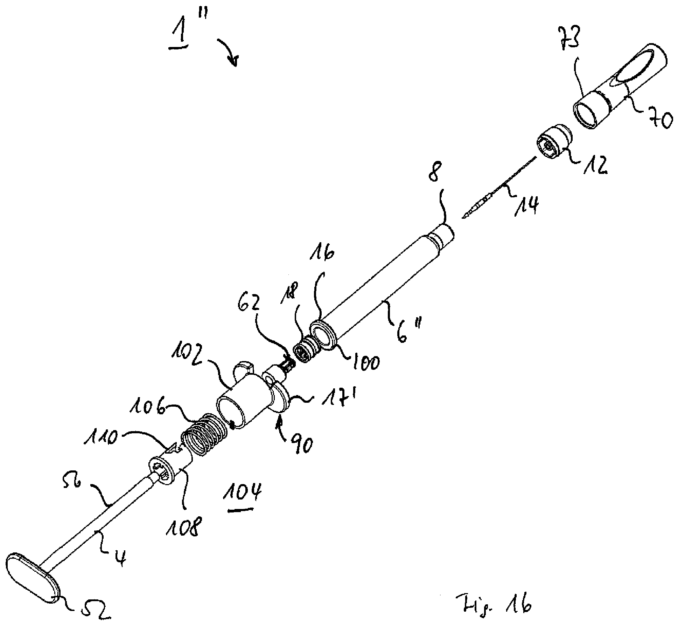

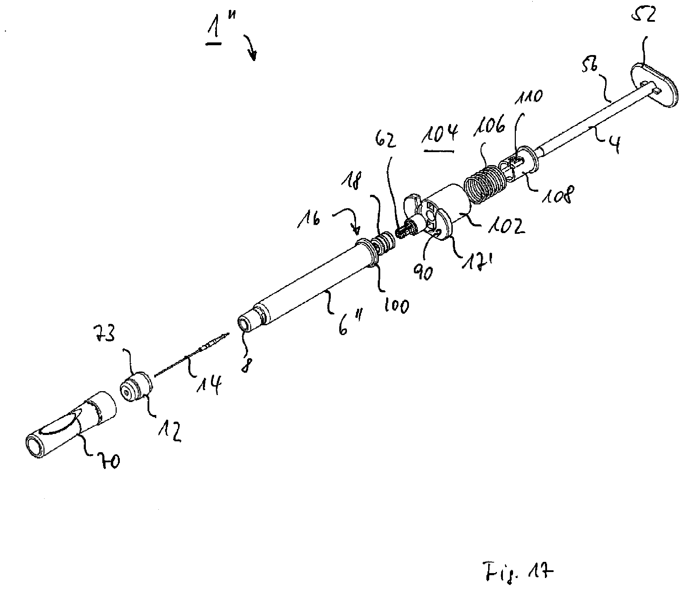

Die zur Bildung eines solchen passiven Retraktionssystems der medizinischen Spritze 1 " vorgesehenen Komponenten sind in den Figs. 16, 17 in verschiedenen Blickrichtungen jeweils in Explosionsdarstellung gezeigt. In dieser Ausführungsform ist auch der das Spritzengehäuse bildende Hohlkörper 6" alternativ zur vorstehend beschriebenen Bauweise ausgeführt; er umfasst nun eine separate Griffplatte 17', die auf einen an seinem proximalen Ende 16 umlaufenden Rastrand 100 aufgeschoben werden kann. An dieser alternativ ausgeführten Griffplatte 17", die im Übrigen im Ausführungsbeispiel ebenfalls entsprechend der vorstehend beschriebenen Bauweise den RFID-Chip 90 trägt, ist auf ihrer dem Hohlkörper 6" abgewandten Seite das Außengehäuse 102 des Retraktionsmechanismus 104 angeformt. Die medizinische Spritze 1 ", die im Übrigen ebenfalls analog zur oben beschriebenen Bauweise mit dem Originalitätsverschluss 70 versehen ist, ist durch den Retraktionsmechanismus 104 mit dem erwünschten passiven Nadelschutz versehen. Damit wird bezweckt, dass nach der Benutzung der Spritze 1 ", also nach der Ausgabe des im das Spritzengehäuse bildenden Hohlkörper 6" vorgehaltenen Wirkstoffs über die Nadel 14, diese in das Spritzengehäuse derart hineingezogen wird, dass sie vom Spritzengehäuse vollständig umschlossen wird. Damit soll eine unbeabsichtigte Berührung der benutzten Nadel 14, beispielsweise durch Hilfs- oder Pflegepersonal, und somit das Verletzungs- und Kontaminationsrisiko besonders gering gehalten oder möglichst ganz ausgeschlossen werden.

Analog zu der aus der EP 3 738 628 A1 bekannten Ausführung ist der Retraktionsmechanismus 104 nach dem Funktionsprinzip aufgebaut, dass eine unter Vorspannung stehenden komprimierte Sprungfeder 106 mittels eines Verriegelungselements 108 arretiert wird. Das Verriegelungselement 108 wird dabei zu gegebener Zeit in Folge des Vorschubs des Betätigungsstößels 4 entriegelt und gibt die Sprungfeder 106 frei, so dass diese sich entspannen kann und dabei den Betätigungsstößel 4 zurückschiebt.

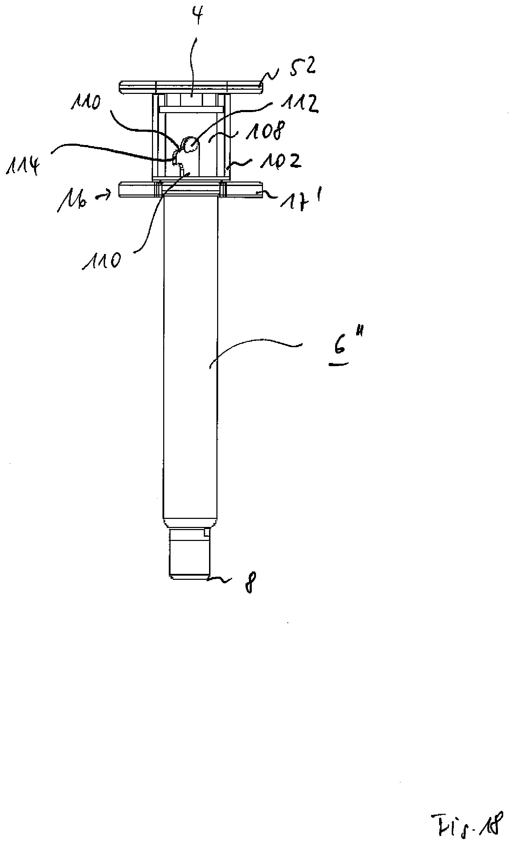

Als eigenständig erfinderisch angesehen wird hierbei die Ausführung des Verriegelungsmechanismus für die Sprungfeder 106. Der Verriegelungsmechanismus beruht dabei im Wesentlichen auf dem Zusammenwirken einer Anzahl von an der Innenoberfläche des Außengehäuses 102 angeordneten Steuerpins mit jeweils einer zugeordneten Führungskulisse 110 im Außenmantel des Verriegelungselements 108. Wie der Darstellung in seitlicher Ansicht gemäß Fig. 18 gut entnehmbar ist, greift der Steuerpin 112 in die in den Außenmantel des Verriegelungselements 108 eingebrachte Führungskulisse 110 ein. Seitlich weist die Führungskulisse 110 eine Ausbuchtung 114 auf, in die der Steuerpin 112 hineingedreht werden kann. Die Ausbuchtung 114 ist dabei derart konturiert, dass der Steuerpin 112 nach unten, also zum Hohlkörper 6" hin, formschlüssig gehalten ist und somit nicht ausweichen kann. In diesem Zustand, in dem die innen liegende Sprungfeder 106 komprimiert ist, ist das System somit arretiert und die Sprungfeder 106 kann sich nicht entspannen.

Die Entriegelung kann dann erfolgen, indem der Steuerpin 112 durch geeignete Ansteuerung seitlich gedreht und aus der Ausbuchtung 114 herausbewegt wird. Damit kommt er über einem nach unten offenen Kanal 116 der Führungskulisse 110 zu liegen, so dass er nunmehr relativ zum Verriegelungselement 108 nach unten hin ausweichen kann und durch die Führungskulisse 110 nicht mehr formschlüssig blockiert ist. In dieser Position kann somit das Verriegelungselement 108 ohne Blockade durch den Steuerpin 112 durch die Sprungfeder 106 relativ zum Außengehäuse 102 nach oben hin, also weg vom Hohlkörper 6", verschoben werden, wobei sich die Sprungfeder 106 entspannt.

In Reaktion darauf, also auf die Auslösung der Sprungfeder 106, entspannt diese sich also und bewegt dabei die Drückerplatte 52 des Betätigungsstößels 4 vom durch den Hohlkörper 6" gebildeten Spritzenkörper weg. Damit wird auch das mit dem Schaft 56 des Betätigungsstößels 4 verbundene Nadellager 62, in dem sich in dieser Phase bereits die Nadel 14 befindet, mitgenommen und innerhalb des Hohlkörpers 6" vom distalen Ende 8 weg zum proximalen Ende 16 hin gezogen. Dabei nimmt er seinerseits die eingefasste Nadel 14 mit und zieht diese in den Hohlkörper 6" hinein, so dass diese im Endzustand vollständig innerhalb des Hohlkörpers 6" positioniert ist.

Ein bedeutsamer und als erfinderisch angesehener Aspekt ist hierbei, dass die zur Durchführung der Retraktionsbewegung der Nadel 14 in den Hohlkörper 6" hinein vorgesehene Sprungfeder 106 oberhalb der Griffplatte 17' im Bereich des Schafts 56 des Betätigungsstößels 4 angeordnet ist und über dessen Drückerplatte 52 auf den Betätigungsstößel 4 wirkt. Im Gegensatz zu üblichen Retraktionssystemen, bei denen die Retraktionsfeder im Bereich der Spitze oder des distalen Endes der Spritze innerhalb des Spritzenkörpers angeordnet ist und direkt auf die Nadel oder deren Lagerkörper einwirkt, bedeutet die nunmehr erfindungsgemäß vorgesehene Auslegung, dass die Nadel 14 bei der Retraktion gemeinsam mit dem Betätigungsstößel 4 und insbesondere dessen Drückerplatte 52 bewegt wird, so dass der Benutzer oder Bediener anhand der Position der Drückerplatte 52 relativ zur Griffplatte 17' die Position und vor allem die Positionsänderung der Nadel 14 erkennen kann. Diese Erkennung ist optisch möglich, vor allem aber auch haptisch.

Mit anderen Worten erzeugt die Retraktionsbewegung, ähnlich wie bei einem Betätigungsknopf eines Kugelschreibers, eine für den Benutzer beispielsweise mit dem Daumen spürbare Bewegung der Drückerplatte 52. Damit kann der Benutzer aus der mit dem Daumen spürbaren Bewegung der Drückerplatte 52 einerseits darauf schließen, dass die Nadel 14 nunmehr eingezogen ist und somit im Bereich des distalen Endes 8 der Spritze 1 " keine Verletzungs- oder Kontaminationsgefahr mehr besteht. Andererseits kann er daraus aber auch schließen, dass der Wirkstoff nunmehr vollständig verabreicht wurde, ohne dies beispielsweise optisch überwachen zu müssen. Die Spritze 1 " ermöglicht somit eine deutlich vereinfachte Handhabung bei der Verabreichung des Wirkstoffs.

### Bezugszeichenliste

- 1, 1', 1": Medizinische Spritze
- 4: Betätigungsstößel
- 6, 6', 6": Hohlkörper
- 8: distales Ende
- 10: Verbindungselement
- 12: Nadelhalter
- 14: Hohlnadel
- 16: proximales Ende
- 17, 17': Griffplatte
- 18: Stopfen
- 20: Außengewinde
- 22: Rastkante
- 24: Innenkanal
- 30: Lagermuffe
- 32: Kontaktfläche
- 34: Innengewinde
- 36: Innensechskant
- 38: Fixierlippe
- 40: Nadelrohr
- 42,44: Nadelspitze
- 46: Kunststoffmantel
- 48: Haltenut
- 50: Rastkante
- 52: Drückerplatte
- 54: Greifbügel
- 56: Schaft
- 58, 60: Stellring
- 62: Nadellager
- 64: Freiraum
- 70: Schutzkappe
- 72: Verbindungssteg
- 73: Sicherungsring
- 74: Kappengehäuse
- 76: Bedienfeld
- 80: Rastaufnahme
- 82: Ringsegmenten
- 84: Rasthaken
- 86: Oberseite
- 90: RFID-Chip
- 91: Ausformung
- 92: Fingerauflage
- 100: Rastrand
- 102: Außengehäuse
- 104: Retraktionsmechanismus
- 106: Sprungfeder
- 108: Verriegelungselement
- 110: Führungskulisse
- 112: Steuerpin
- 114: Ausbuchtung
- 116: Kanal

## Patentansprüche

1. Medizinische Spritze (1) mit einem das Spritzengehäuse bildenden, zur Aufnahme eines medizinischen Wirkstoffs vorgesehenen Hohlkörper (6), an dessen distalem Ende (8) ein Verbindungselement (10) angeformt ist, an dem ein Nadelhalter (12) für eine zur Injektion des Wirkstoffs vorgesehene Hohlnadel (14) befestigbar ist, wobei das Verbindungselement (10) einen zur Verbindung mit dem Nadelhalter (12) vorgesehenen Innenkanal (24) aufweist, der einen sich zum distalen Ende (8) hin kontinuierlich aufweitenden Innenquerschnitt aufweist.

2. Medizinische Spritze (1) nach Anspruch 1, bei der der Innenkanal (24) in der Art eines sich zum distalen Ende (8) hin aufweitenden Konus ausgestaltet ist.

3. Medizinische Spritze (1) nach Anspruch 2, bei der der durch den Innenkanal (24) gebildete Konus einen Konuswinkel etwa 2° beträgt.

4. Medizinische Spritze (1), insbesondere nach einem der Ansprüche 1 bis 3, mit einem das Spritzengehäuse bildenden, zur Aufnahme eines medizinischen Wirkstoffs vorgesehenen Hohlkörper (6), an dessen proximalem Ende (16) eine Griffplatte (17) angeformt ist, an der ein RFID-Chip (90) angeordnet ist.

5. Medizinische Spritze (1), insbesondere nach einem der Ansprüche 1 bis 4, mit einem Betätigungsstößel (4) und mit einem das Spritzengehäuse bildenden, zur Aufnahme eines medizinischen Wirkstoffs vorgesehenen Hohlkörper (6), wobei am Schaft (56) des Betätigungsstößels (4) eine Anzahl von Anschlag- und Rastelementen angeordnet ist.

6. Medizinische Spritze (1) nach Anspruch 5, deren Rastelemente als um den Schaft (56) umlaufende Stellringe (58, 60) ausgeführt sind.
